# EUROPEAN PATENT APPLICATION

(11) **EP 3 459 596 A1**
(43) Date of publication of application: **27.03.2019**
(21) Application number: 17193098.5
(22) Date of filing: 26.09.2017
(51) Int. Cl.: A61N 7/02, A61N 7/00, A61B 5/00, A61B 5/01, G01R 33/48, A61B 90/00

(54) **POWER ADJUSTMENT IN MAGNETIC RESONANCE GUIDED HIGH INTENSITY FOCUSED ULTRASOUND**

(71) Applicant: Koninklijke Philips N.V., 5656 AE Eindhoven (NL)
(72) Inventor: HEIJMAN, Edwin, 5656 AE Eindhoven (NL); SEBEKE, Lukas, 5656 AE Eindhoven (NL); STARUCH, Robert Michael, 5656 AE Eindhoven (NL)
(74) Representative: de Haan, Poul Erik

(57) **Abstract**

The invention provides for a medical instrument (100) comprising: a magnetic resonance imaging system (102) and a high intensity focused ultrasound system (122) comprising an ultrasonic transducer (126). The ultrasonic transducer being configured for simultaneously heating multiple sonication volumes (138, 138', 138"). Execution of machine executable instructions (180) cause a processor (174) to: receive (300) sonication commands (188) configured for controlling the ultrasonic transducer to sonicate the multiple sonication volumes, receive (302) an acoustic power setting (190) for scaling the acoustic power, wherein the relative power of the electrical voltage supplied to each of the multiple ultrasonic transducer elements is scaled using the acoustic power setting, and
control (304) the high intensity focused ultrasound system to sonicate the multiple sonication volumes. During the sonication, the processor repeatedly: acquires (306), and reconstructs (308), and displays 310) a thermal map (194) of the multiple sonication volumes. During the sonication, the processor further repeatedly: receives (312) a pause command (196) which causes the processor to pause the sonication of the multiple sonication volumes; receives (314) a resume command (196) which causes the processor to resume the sonication of the multiple sonication volumes; and receives (316) a change power command which causes the processor to change (320) the relative power of the electrical voltage supplied to each of the multiple ultrasonic transducer elements proportionally by a value specified in the change power command, wherein an increase of the relative power of the electrical voltage supplied to the ultrasonic transducer above a predetermined value (198) is only performed if (318) the sonication of the multiple sonication volumes is paused.

## Description

### FIELD OF THE INVENTION

The invention relates to magnetic resonance guided high intensity focused ultrasound.

### BACKGROUND OF THE INVENTION

Ultrasound from a focused ultrasonic transducer can be used to selectively treat regions within the interior of the body. Ultrasonic waves are transmitted as high energy mechanical vibrations. These vibrations induce tissue heating as they are damped, and they can also lead to cavitation. Both tissue heating and cavitation can be used to destroy tissue in a clinical setting. Ultrasonic treatments can be used to ablate tissue and to kill regions of cancer cells selectively. This technique has been applied to the treatment of uterine fibroids, and has reduced the need for hysterectomy procedures.

At lower power levels, ultrasound can be used to warm up tissue instead of ablating it. Raising tissue temperatures to elevated, but non-ablating levels is known as hyperthermia (ranging from 40°C-45°C). Hyperthermia has been shown to make heated tissue more sensitive to radiation therapy, chemotherapy as well as chemo radiation therapy. Hyperthermia can also be used to locally release drugs such as chemotherapeutic agents or antibiotics. Hyperthermia can therefore be a useful clinical tool.

International patent application publication WO 2014/057388 A1 discloses a mild hyperthermia treatment apparatus that includes an imager, which generates a planning image and temperature maps of a target region. An array of ultrasonic transducer drivers individually drives ultrasonic transducers of a phased array of ultrasonic transducers. One or more processors or units receive a target temperature profile, and calculate power, frequency, and relative phase for the transducer drivers to drive the phased array of ultrasonic transducers to generate a multi-foci sonication pattern configured to heat the target region with the target temperature profile while limiting peak acoustic pressures. During treatment, the imager generates a series of temperature maps which the one or more processors or units compare with the target temperature profile and, based on the comparison, adjust the power and relative phase with which the transducer drivers drive the ultrasonic transducers.

### SUMMARY OF THE INVENTION

The invention provides for a medical instrument, a computer program product and a method in the independent claims. Embodiments are given in the independent claims.

When performing hyperthermia, it is important not to heat the tissue too high, or the tissue perfusion will be stopped and/or the tissue could die. Additionally, it is difficult to set the proper power level, because the heating rate of tissues depends upon many factors, which can vary from subject to subject. For this reason, when physicians are performing hyperthermia using a high intensity focused ultrasound (HIFU) system, they tend to be very conservative when setting power levels so as not to injure the subject. Typically, a physician will set a power level and then start the sonication to induce hyperthermia. If the power level is too low to induce sufficient heating, the physician can abort the sonication to increase the output power limit. This also holds if the chosen power is too high, which can result in temperature overshoots, necessitating a lowering of the power output limit. After aborting a sonication however, the physician then can wait for a period of time until the heated volumes have returned to normal body temperature before starting the process again. The physician may repeat this process multiple times before the proper sonication power level is found. As the waiting time can be as long as 10 or 15 minutes, a huge amount of time might be wasted and the HIFU system is inefficiently used.

Embodiments of the invention may address the above issues by providing an additional control for the physician, to send a command to the HIFU system to increase or decrease the power level. However, additional safeguards are put in place. Before the HIFU system is instructed to change the power level, the new power level is compared to a predetermined level to see if the change in the power level is allowed. If the new acoustic power level is above the predetermined level, then the sonication must be paused before the new power level is set. In different examples, the predetermined level could be determined differently and are described below in the embodiments. The requirement of a pause in the sonication prevents the power level from inadvertently being increased too much. The sonication can then be resumed using a resume command. The increase in the power was achieved and the sonication was resumed avoiding the delay waiting for the sonicated tissue to return to body temperature. The pausing of the sonication also provides a verification that the increase in the acoustic power level was intended.

In one aspect, the invention provides for a medical instrument comprising a magnetic resonance imaging system for acquiring magnetic resonance thermometry data from a subject within an imaging zone. Magnetic resonance thermometry data as used herein encompasses magnetic resonance data that may be used for determining a spatial temperature distribution within a subject. The medical instrument further comprises a high-intensity focused ultrasound system comprising an ultrasonic transducer. The ultrasonic transducer comprises multiple ultrasonic transducer elements, the ultrasonic transducer being configured for simultaneously sonicating multiple sonication volumes within the imaging zone. The simultaneous sonication of the multiple sonication volumes may be interpreted in different ways depending upon the configuration of the ultrasonic transducer. In one example, the multiple sonication volumes are all sonicated at the same time. For example, the transducer elements may focus into multiple sonication volumes. In other examples, the focus of the high-intensity focused ultrasound system is adjustable either electronically (by altering the relative phases of the ultrasound elements) and/or mechanically. The simultaneous heating of the multiple sonication volumes is then performed by rapidly and systematically shifting the focus of the ultrasonic transducer. Each of the sonication volumes are then maintained at a therapeutic temperature simultaneously, although ultrasonic energy is applied to the sonication volumes consecutively.

The medical instrument further comprises memory containing machine-executable instructions and thermometry pulse sequence commands. The thermometry pulse sequence commands are configured for acquiring magnetic resonance thermometry data according to a magnetic resonance imaging thermometry protocol. The medical instrument further comprises a processor for controlling the medical instrument and a display.

The machine-executable instructions cause the processor to receive sonication commands configured for controlling the ultrasonic transducer to sonicate the multiple sonication volumes. The sonication commands are configured for controlling at least the relative power supplied to each of the multiple ultrasonic transducer elements. The sonication commands may also contain the location of the focus or foci of the focused ultrasound beam. The sonication commands could for example be received from a user interacting with a graphical user interface on the display where a scout or preliminary magnetic resonance image was used for targeting.

The machine-executable instructions may also cause the processor to perform a calibration or preliminary measurements for the magnetic resonance imaging thermometry protocol.

The machine-executable instructions further cause the processor to receive an acoustic power setting. The relative power of the electrical voltage supplied to each of the multiple ultrasonic transducer elements is scaled using the acoustic power setting. Before sonication begins, the exact absorption properties of a subject's tissue are not known. The acoustic power setting is used effectively to scale the relative power to supply to each of the multiple ultrasonic transducer elements.

Execution of the machine-executable instructions further cause the processor to control the high-intensity focused ultrasound system with the sonication commands to sonicate the multiple sonication volumes. During the execution of the sonication commands the machine-executable instructions repeatedly cause the processor to control the magnetic resonance imaging system with the thermometry pulse sequence commands to acquire the magnetic resonance thermometry data. This step here may be continually repeated even if the sonication is paused. Execution of the machine-executable instructions repeatedly cause the processor to reconstruct a thermal map of the imaging zone using the magnetic resonance thermometry data once the sonication commands have begun to be executed. The reconstruction of the thermal map may be performed repeatedly even if the sonication is paused.

Execution of the machine-executable instructions further cause the processor to display the thermal map on the display. The displaying of the thermal map on the display may be performed repeatedly even if the sonication is later paused. During the sonication of the multiple sonication volumes, execution of the machine-executable instructions repeatedly cause the processor to receive a pause command which causes the processor to pause the sonication of the multiple sonication volumes. The other steps may be repeatedly performed even if the sonication is paused. Execution of the machine-executable instructions further cause the processor to receive a resume command which causes the processor to resume the sonication of the multiple sonication volumes. This step may be performed during a pause of the sonication.

Execution of the machine-executable instructions further cause the processor during the sonication to receive a change power command which causes the processor to change the relative power of the electrical voltage supplied to each of the multiple ultrasonic transducer elements proportionally by a value specified in the change power command. For example, the change power command may be given in terms of absolute power or a scaling factor relative to the acoustic power setting. An increase of the relative power of the electrical voltage supplied to each of the multiple ultrasonic transducer elements above a predetermined value is only performed if the sonication of the multiple sonication volumes is paused. That is to say an increase of the power in the electrical voltage supplied to each multiple ultrasonic transducer may only happen if the sonication of the multiple sonication volumes is paused and also if the increase is below a predetermined value. The predetermined value may be different in different examples. If, for example, the predetermined value is at the current power level, any increase requires the pausing of the sonication. In another example, the predetermined value is the acoustic power setting or some scale factor of the acoustic power setting.

This embodiment may have the benefit of providing for a more rapid sonication of the multiple sonication volumes. It may also be beneficial because it provides an additional level of safety such that the change power command does not inadvertently increase the power to a level which may harm the subject.

In another embodiment, execution of the machine-executable instructions further causes the processor to render a graphical user interface on the display. The graphical user interface comprises a power adjustment control configured for generating the change power command. This embodiment may provide an easy way for an operator to adjust the scaling factor used to scale the relative power supply to each of the multiple ultrasonic transducer elements. The power adjustment control may for example be a slider, knob, text input box, or other graphical user input control.

In another embodiment, the graphical user interface further comprises at least one button for generating the pause command and/or the resume command. For example in some embodiments there may be a separate button for each one.

The combination of the thermal map on the display in conjunction with the power adjustment control and the at least one button for generating the pause command and/or the resume command provide for an effective feedback control system for manually adjusting the medical instrument during the sonication.

In another embodiment, the high-intensity focused ultrasound system is configured for only performing hyperthermia within the multiple sonication volumes. For example, the high-intensity focused ultrasound system could have its maximum power limited or the thermal imaging could be used to automatically reduce the power if it goes outside of the temperature range for which hyperthermia is normally considered.

In another embodiment, the high-intensity focused ultrasound system is configured for heating the multiple sonication volumes to temperatures within a predetermined temperature range.

In another embodiment, the predetermined temperature range is between 40°C and 45°C.

In another embodiment, the predetermined temperature range if between 41°C and 43°C.

In another embodiment, the predetermined value is the acoustic power setting. In this embodiment, the change power command is not able to increase the acoustic power above its initial setting unless the sonication is paused. However, the sonication values can be lowered to below the acoustic power setting and then raised back up to the 100% setting without pausing. This may be effective because typically, when hyperthermia sonication's are performed, the physician will start out at an acoustic power setting which the physician or technician knows to be safe. There is therefore no need to pause the multiple sonication volumes when returning to the initial acoustic power setting.

In another embodiment, the predetermined value is equivalent to the current acoustic power level. In this embodiment if any increase in the acoustic power is desired the sonication's must be paused. This may provide for a safer system in that the pausing requires the operator to intentionally increase the acoustic power.

In another embodiment, execution of the machine-executable instructions causes aborting of the sonication of the multiple sonication volumes if a pause in the sonication is longer than a predetermined duration. This may be beneficial because it may prevent the multiple sonication volumes from cooling too much during the adjustment of the acoustic power value.

In another embodiment, execution of the machine-executable instructions causes the aborting of the sonication of the multiple sonication volumes if a temperature within the multiple sonication volumes as determined by the thermal map drops below a predetermined lower threshold. If there is a temperature below a predetermined lower threshold this may indicate that the sonication is not proceeding properly. This may for example be performed after a certain delay which enables for initial heating of the multiple sonication volumes.

In another embodiment, execution of the machine-executable instructions causes aborting of the sonication of the multiple sonication volumes if a temperature heating rate within one of the multiple sonication volumes is below a predetermined heating rate. This may also be beneficial because it may indicate that the sonication of the multiple sonication volumes is not functioning properly.

In another embodiment, execution of the machine-executable instructions further causes aborting of the sonication of the multiple sonication volumes if a temperature within the multiple sonication volumes as determined by the thermal map is above a predetermined upper threshold. This may be beneficial because it may help prevent burning of the subject.

In another embodiment, execution of the machine-executable instructions may further cause aborting of the sonication of the multiple sonication volumes if motion of the subject is detected. This motion may for example be above a certain threshold or degree of motion of the subject. The motion of the subject could be for example detected using various methods such as pressure sensors in a table, cameras or other observational techniques, or even routinely made magnetic resonance images. For example, the thermometry pulse sequence commands may be used to detect motion of the subject. Due to thermal inertia, the regions being heated within the subject will tend to stay in one location if the subject is not moving. If the warm spots in the thermal maps begin to wander or move abruptly this may indicate motion of the subject.

In another embodiment, during a pause of the sonication of the multiple sonication volumes the following steps are repeatedly performed: controlling the magnetic resonance imaging system with the thermometry pulse sequence commands to acquire the magnetic resonance thermometry data and reconstructing a thermal map of the imaging zone using the magnetic resonance thermometry data.

During the pausing of the sonication of the multiple sonication volumes the step of displaying the thermal map on the display may also be periodically performed or updated or refreshed.

In another aspect, the invention provides for a method of controlling a medical instrument. The medical instrument comprises a magnetic resonance imaging system for acquiring magnetic resonance thermometry data from a subject within an imaging zone. The medical instrument further comprises a high-intensity focused ultrasound system comprising an ultrasonic transducer. The ultrasonic transducer comprises multiple ultrasonic transducer elements. The ultrasonic transducer being configured for simultaneously heating multiple sonication volumes within the imaging zone. The medical instrument further comprises a display.

The method comprises receiving sonication commands configured for controlling the ultrasonic transducer to sonicate the multiple sonication volumes. These sonication commands are configured for controlling at least the relative power supplied to each of the multiple ultrasonic transducer elements. The method further comprises receiving an acoustic power setting. The relative power of the electrical voltage supplied to each of the multiple transducer elements is scaled using the acoustic power setting.

The method further comprises controlling the high-intensity focused ultrasound system with the sonication commands to sonicate the multiple sonication volumes. The method further comprises controlling the magnetic resonance imaging system with thermometry pulse sequence commands to acquire magnetic resonance thermometry data. The thermometry pulse sequence commands are configured for acquiring the magnetic resonance thermometry data according to a magnetic resonance imaging thermometry protocol. The method further comprises reconstructing a thermal map of the imaging zone using the magnetic resonance thermometry data. The method further comprises displaying the thermal map on the display.

The method further comprises receiving a pause command which causes the processor to pause the sonication of the multiple sonication volumes. The method further comprises receiving a resume command which causes the processor to resume the sonication of the multiple sonication volumes. The method further comprises receiving a change power command which causes the processor to change the relative power of the electrical voltage supplied to each of the multiple ultrasonic transducer elements proportionally by a value specified in the change power command. An increase of the relative power of the electrical voltage supplied to each of the multiple ultrasonic transducer elements above a predetermined value is only performed if the sonication of the multiple sonication volumes is paused. The advantages of this method have been previously discussed.

In another aspect, the invention provides for a computer program product comprising machine-executable instructions for execution by a processor controlling a medical instrument. The medical instrument comprises a magnetic resonance imaging system for acquiring magnetic resonance thermometry data from a subject within an imaging zone. The medical instrument further comprises a high-intensity focused ultrasound system comprising an ultrasonic transducer. The ultrasonic transducer comprises multiple ultrasonic transducer elements. The ultrasonic transducer being configured for simultaneously sonicating multiple sonication volumes within the imaging zone. The medical instrument further comprises a display. Execution of the machine-executable instructions further causes the processor to receive sonication commands configured for controlling the ultrasonic transducer to sonicate the multiple sonication volumes. The sonication commands are configured for controlling at least the relative power supplied to each of the multiple ultrasonic transducer elements. Execution of the machine-executable instructions further cause the processor to receive an acoustic power setting. The relative power of the electrical voltage supplied to each of the multiple ultrasonic transducer elements is scaled using the acoustic power setting. Execution of the machine-executable instructions further cause the processor to control the high-intensity focused ultrasound system with the sonication commands to sonicate the multiple sonication volumes.

Execution of the machine-executable instructions further cause the processor to control the magnetic resonance imaging system with the thermometry pulse sequence commands to acquire the magnetic resonance thermometry data. The thermometry pulse sequence commands are configured for acquiring the magnetic resonance thermometry data according to a magnetic resonance imaging thermometry protocol. Execution of the machine-executable instructions further cause the processor to reconstruct a thermal map of the imaging zone using the magnetic resonance thermometry data. Execution of the machine-executable instructions further cause the processor to display the thermal map on the display.

Execution of the machine-executable instructions further cause the processor to receive a pause command which causes the processor to pause the sonication of the multiple sonication volumes. Execution of the machine-executable instructions further cause the processor to receive a resume command which causes the processor to resume the sonication of the multiple sonication volumes. Execution of the machine-executable instructions further cause the processor to receive a change power command which causes the processor to change the relative power of the electrical voltage supplied to each of the multiple ultrasonic transducer elements proportionally by a value specified in the change power command. An increase of the relative power of the electrical voltage supplied to each of the multiple ultrasonic transducer elements above a predetermined value is only performed if the sonication of the multiple sonication volumes is paused.

In another embodiment, execution of the machine-executable instructions further causes the processor to render a graphical user interface on the display. The graphical user interface comprises a power adjustment control configured for generating the change power command.

In another embodiment, the graphical user interface further comprises at least one button for generating the pause command and/or the resume command.

It is understood that one or more of the aforementioned embodiments of the invention may be combined as long as the combined embodiments are not mutually exclusive.

As will be appreciated by one skilled in the art, aspects of the present invention may be embodied as an apparatus, method or computer program product. Accordingly, aspects of the present invention may take the form of an entirely hardware embodiment, an entirely software embodiment (including firmware, resident software, microcode, etc.) or an embodiment combining software and hardware aspects that may all generally be referred to herein as a "circuit," "module" or "system." Furthermore, aspects of the present invention may take the form of a computer program product embodied in one or more computer readable medium(s) having computer executable code embodied thereon.

Any combination of one or more computer readable medium(s) may be utilized. The computer readable medium may be a computer readable signal medium or a computer readable storage medium. A 'computer-readable storage medium' as used herein encompasses any tangible storage medium which may store instructions which are executable by a processor of a computing device. The computer-readable storage medium may be referred to as a computer-readable non-transitory storage medium. The computer-readable storage medium may also be referred to as a tangible computer readable medium. In some embodiments, a computer-readable storage medium may also be able to store data which is able to be accessed by the processor of the computing device. Examples of computer-readable storage media include, but are not limited to: a floppy disk, a magnetic hard disk drive, a solid state hard disk, flash memory, a USB thumb drive, Random Access Memory (RAM), Read Only Memory (ROM), an optical disk, a magneto-optical disk, and the register file of the processor. Examples of optical disks include Compact Disks (CD) and Digital Versatile Disks (DVD), for example CD-ROM, CD-RW, CD-R, DVD-ROM, DVD-RW, or DVD-R disks. The term computer readable-storage medium also refers to various types of recording media capable of being accessed by the computer device via a network or communication link. For example a data may be retrieved over a modem, over the internet, or over a local area network. Computer executable code embodied on a computer readable medium may be transmitted using any appropriate medium, including but not limited to wireless, wire line, optical fiber cable, RF, etc., or any suitable combination of the foregoing.

A computer readable signal medium may include a propagated data signal with computer executable code embodied therein, for example, in baseband or as part of a carrier wave. Such a propagated signal may take any of a variety of forms, including, but not limited to, electro-magnetic, optical, or any suitable combination thereof. A computer readable signal medium may be any computer readable medium that is not a computer readable storage medium and that can communicate, propagate, or transport a program for use by or in connection with an instruction execution system, apparatus, or device.

'Computer memory' or 'memory' is an example of a computer-readable storage medium. Computer memory is any memory which is directly accessible to a processor. 'Computer storage' or 'storage' is a further example of a computer-readable storage medium. Computer storage may be any volatile or non-volatile computer-readable storage medium.

A 'processor' as used herein encompasses an electronic component which is able to execute a program or machine executable instruction or computer executable code. References to the computing device comprising "a processor" should be interpreted as possibly containing more than one processor or processing core. The processor may for instance be a multi-core processor. A processor may also refer to a collection of processors within a single computer system or distributed amongst multiple computer systems. The term computing device should also be interpreted to possibly refer to a collection or network of computing devices each comprising a processor or processors. The computer executable code may be executed by multiple processors that may be within the same computing device or which may even be distributed across multiple computing devices.

Computer executable code may comprise machine executable instructions or a program which causes a processor to perform an aspect of the present invention. Computer executable code for carrying out operations for aspects of the present invention may be written in any combination of one or more programming languages, including an object oriented programming language such as Java, Smalltalk, C++ or the like and conventional procedural programming languages, such as the C programming language or similar programming languages and compiled into machine executable instructions. In some instances the computer executable code may be in the form of a high level language or in a pre-compiled form and be used in conjunction with an interpreter which generates the machine executable instructions on the fly.

The computer executable code may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider).

Aspects of the present invention are described with reference to flowchart illustrations and/or block diagrams of methods, apparatus (systems) and computer program products according to embodiments of the invention. It is understood that each block or a portion of the blocks of the flowchart, illustrations, and/or block diagrams, can be implemented by computer program instructions in form of computer executable code when applicable. It is further understood that, when not mutually exclusive, combinations of blocks in different flowcharts, illustrations, and/or block diagrams may be combined. These computer program instructions may be provided to a processor of a general purpose computer, special purpose computer, or other programmable data processing apparatus to produce a machine, such that the instructions, which execute via the processor of the computer or other programmable data processing apparatus, create means for implementing the functions/acts specified in the flowchart and/or block diagram block or blocks.

These computer program instructions may also be stored in a computer readable medium that can direct a computer, other programmable data processing apparatus, or other devices to function in a particular manner, such that the instructions stored in the computer readable medium produce an article of manufacture including instructions which implement the function/act specified in the flowchart and/or block diagram block or blocks.

The computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts specified in the flowchart and/or block diagram block or blocks.

A 'user interface' as used herein is an interface which allows a user or operator to interact with a computer or computer system. A 'user interface' may also be referred to as a 'human interface device.' A user interface may provide information or data to the operator and/or receive information or data from the operator. A user interface may enable input from an operator to be received by the computer and may provide output to the user from the computer. In other words, the user interface may allow an operator to control or manipulate a computer and the interface may allow the computer indicate the effects of the operator's control or manipulation. The display of data or information on a display or a graphical user interface is an example of providing information to an operator. The receiving of data through a keyboard, mouse, trackball, touchpad, pointing stick, graphics tablet, joystick, gamepad, webcam, headset, pedals, wired glove, remote control, and accelerometer are all examples of user interface components which enable the receiving of information or data from an operator.

A 'hardware interface' as used herein encompasses an interface which enables the processor of a computer system to interact with and/or control an external computing device and/or apparatus. A hardware interface may allow a processor to send control signals or instructions to an external computing device and/or apparatus. A hardware interface may also enable a processor to exchange data with an external computing device and/or apparatus. Examples of a hardware interface include, but are not limited to: a universal serial bus, IEEE 1394 port, parallel port, IEEE 1284 port, serial port, RS-232 port, IEEE-488 port, bluetooth connection, wireless local area network connection, TCP/IP connection, ethernet connection, control voltage interface, MIDI interface, analog input interface, and digital input interface.

A 'display' or 'display device' as used herein encompasses an output device or a user interface adapted for displaying images or data. A display may output visual, audio, and or tactile data. Examples of a display include, but are not limited to: a computer monitor, a television screen, a touch screen, tactile electronic display, Braille screen,

Cathode ray tube (CRT), Storage tube, Bi-stable display, Electronic paper, Vector display, Flat panel display, Vacuum fluorescent display (VF), Light-emitting diode (LED) display, Electroluminescent display (ELD), Plasma display panel (PDP), Liquid crystal display (LCD), Organic light-emitting diode display (OLED), a projector, and Head-mounted display.

Medical imaging data is defined herein as two or three dimensional data that has been acquired using a medical imaging system. A medical imaging system is defined herein as an apparatus adapted for acquiring information about the physical structure of a patient and construct sets of two dimensional or three dimensional medical imaging data. Medical imaging data can be used to construct visualizations which might be useful for diagnosis by a physician. This visualization can be performed using a computer.

Magnetic Resonance (MR) data is defined herein as being the recorded measurements of radio frequency signals emitted by atomic spins using the antenna of a magnetic resonance apparatus during a magnetic resonance imaging scan. Magnetic resonance data is an example of medical imaging data. A Magnetic Resonance (MR) image is defined herein as being the reconstructed two or three dimensional visualization of anatomic data contained within the magnetic resonance imaging data.

MR thermometry data may be understood as being the recorded measurements of radio frequency signals emitted by atomic spins by the antenna of a Magnetic resonance apparatus during a magnetic resonance imaging scan which contains information which may be used for magnetic resonance thermometry. Magnetic resonance thermometry functions by measuring changes in temperature sensitive parameters. Examples of parameters that may be measured during magnetic resonance thermometry are: the proton resonance frequency shift, the diffusion coefficient, or changes in the T1 and/or T2 relaxation time may be used to measure the temperature using magnetic resonance. The proton resonance frequency shift is temperature dependent, because the magnetic field that individual protons, hydrogen atoms, experience depends upon the surrounding molecular structure. An increase in temperature decreases molecular screening due to the temperature affecting the hydrogen bonds. This leads to a temperature dependence of the proton resonant frequency.

The proton density depends linearly on the equilibrium magnetization. It is therefore possible to determine temperature changes using proton density weighted images.

The relaxation times T1, T2, and T2-star (sometimes written as T2*) are also temperature dependent. The reconstruction of T1, T2, and T2-star weighted images can therefore be used to construct thermal or temperature maps.

The temperature also affects the Brownian motion of molecules in an aqueous solution. Therefore pulse sequences which are able to measure diffusion coefficients such as a pulsed diffusion gradient spin echo may be used to measure temperature.

One of the most useful methods of measuring temperature using magnetic resonance is by measuring the proton resonance frequency (PRF) shift of water protons. The resonant frequency of the protons is temperature dependent. As the temperature changes in a voxel the frequency shift will cause the measured phase of the water protons to change. The temperature change between two phase images can therefore be determined. This method of determining temperature has the advantage that it is relatively fast in comparison to the other methods. The PRF method is discussed in greater detail than other methods herein. However, the methods and techniques discussed herein are also applicable to the other methods of performing thermometry with magnetic resonance imaging.

An 'ultrasound window' as used herein encompasses a window which is able to transmit ultrasonic waves or energy. Typically a thin film or membrane is used as an ultrasound window. The ultrasound window may for example be made of a thin membrane of BoPET (Biaxially-oriented polyethylene terephthalate).

### BRIEF DESCRIPTION OF THE DRAWINGS

In the following preferred embodiments of the invention will be described, by way of example only, and with reference to the drawings in which:
Fig. 1 illustrates an example of a medical instrument;
Fig. 2 illustrates an example of a user interface;
Fig. 3 shows a flow chart which illustrates a method of operating the medical instrument of Fig. 1; and
Fig. 4; shows a flow chart which illustrates a further method of operating the medical instrument of Fig. 1.

### DETAILED DESCRIPTION OF THE EMBODIMENTS

Like numbered elements in these figures are either equivalent elements or perform the same function. Elements which have been discussed previously will not necessarily be discussed in later figures if the function is equivalent.

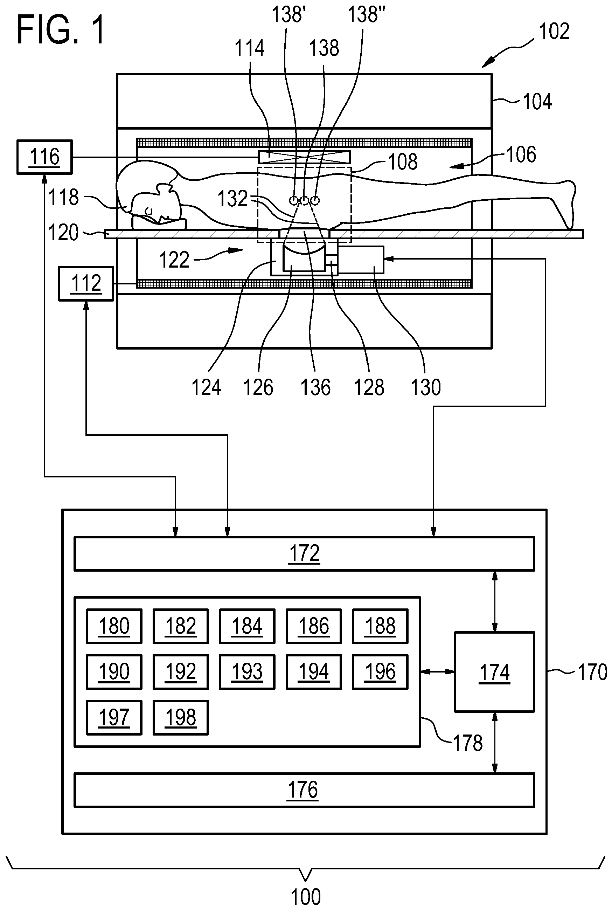

Fig. 1 shows an example of a medical instrument 100. The medical instrument 100 comprises a magnetic resonance imaging system 102 and a high-intensity focused ultrasound system 122. The magnetic resonance imaging system 102 comprises a magnet 104. The magnet 104 is a cylindrical type superconducting magnet with a bore 106 through the center of it. The magnet has a liquid helium cooled cryostat with superconducting coils. It is also possible to use permanent or resistive magnets. The use of different types of magnets is also possible for instance it is also possible to use both a split cylindrical magnet and a so called open magnet. A split cylindrical magnet is similar to a standard cylindrical magnet, except that the cryostat has been split into two sections to allow access to the iso-plane of the magnet, such magnets may for instance be used in conjunction with charged particle beam therapy. An open magnet has two magnet sections, one above the other with a space in-between that is large enough to receive a subject: the arrangement of the two sections area similar to that of a Helmholtz coil. Open magnets are popular, because the subject is less confined. Inside the cryostat of the cylindrical magnet there is a collection of superconducting coils. Within the bore 106 of the cylindrical magnet there is an imaging zone 108 where the magnetic field is strong and uniform enough to perform magnetic resonance imaging.

Within the bore 106 of the magnet there is also a set of magnetic field gradient coils 110 which are used for acquisition of magnetic resonance data to spatially encode magnetic spins within the imaging zone 108 of the magnet 104. The magnetic field gradient coils are connected to a magnetic field gradient coil power supply 112. The magnetic field gradient coils 110 are intended to be representative. Typically magnetic field gradient coils contain three separate sets of coils for spatially encoding in three orthogonal spatial directions. A magnetic field gradient power supply 112 supplies current to the magnetic field gradient coils 110. The current supplied to the magnetic field coils is controlled as a function of time and may be ramped or pulsed.

Adjacent to the imaging zone 108 is a radio-frequency coil 114 for manipulating the orientations of magnetic spins within the imaging zone 108 and for receiving radio transmissions from spins also within the imaging zone. The radio-frequency coil may contain multiple coil elements. The radio-frequency coil may also be referred to as a channel or an antenna. The radio-frequency coil 114 is connected to a radio frequency transceiver 116. The radio-frequency coil 114 and radio frequency transceiver 116 may be replaced by separate transmit and receive coils and a separate transmitter and receiver. It is understood that the radio-frequency coil 114 and the radio-frequency transceiver 116 are representative. The radio-frequency coil 114 is intended to also represent a dedicated transmit antenna and a dedicated receive antenna. Likewise the transceiver 116 may also represent a separate transmitter and receivers.

A subject 118 is shown as reposing on a subject support 120 and is located partially within the imaging zone 108. The example shown in Fig. 1 comprises a high-intensity focused ultrasound system 122. The high-intensity focused ultrasound system comprises a fluid-filled chamber 124. Within the fluid-filled chamber 124 is an ultrasound transducer 126. Although it is not shown in this figure the ultrasound transducer 126 may comprise multiple ultrasound transducer elements each capable of generating an individual beam of ultrasound. This may be used to steer the location of a sonication volume 138 electronically by controlling the phase and/or amplitude of alternating electrical current supplied to each of the ultrasound transducer elements.

The ultrasound transducer 126 is connected to a mechanism 128 which allows the ultrasound transducer 126 to be repositioned mechanically. The mechanism 128 is connected to a mechanical actuator 130 which is adapted for actuating the mechanism 128. The mechanical actuator 130 also represents a power supply for supplying electrical power to the ultrasound transducer 126. In some embodiments the power supply may control the phase and/or amplitude of electrical power to individual ultrasound transducer elements. In some embodiments the mechanical actuator/power supply 130 is located outside of the bore 106 of the magnet 104.

The ultrasound transducer 126 generates ultrasound which is shown as following the path 132. The ultrasound 132 goes through the fluid-filled chamber 124 and through an ultrasound window 134. In this embodiment the ultrasound then passes through a gel pad 136. The gel pad 136 is not necessarily present in all embodiments but in this embodiment there is a recess in the subject support 120 for receiving a gel pad 136. The gel pad 136 helps couple ultrasonic power between the transducer 126 and the subject 118. After passing through the gel pad 136 the ultrasound 132 passes through the subject 118 and is focused to a sonication volume 138. The sonication volume 138 may be moved through a combination of mechanically positioning the ultrasonic transducer 126 and electronically steering the position of the sonication point 138.

Within the imaging zone 108 there can be seen three separate sonication volumes 138, 138', 138". It can be seen from the path of the ultrasound 132 that the ultrasonic transducer 126 is currently focused on the sonication volume 138. The three sonication volumes 138, 138', 138" are however heated simultaneously. The focus of the ultrasound 132 is switched between the various sonication volumes rapidly enough so that they are heated simultaneously. This switching of the path of the ultrasound 132 may be accomplished by either mechanical means 128 used to shift the focus of the ultrasonic transducer 126 or to change the path of the ultrasound 132 electronically. Electronic steering of the path of the ultrasound 132 can be controlled by changing the relative phases and/or amplitudes of individual elements of the ultrasound transducer 126.

The magnetic field gradient coil power supply 112, the high-intensity focused ultrasound system 122, and the transceiver 116 are shown as being connected to a hardware interface 172 of a computer system 170. The computer system 170 also comprises a processor 174. The processor 174 may actually represent more than one processor and may also represent processors distributed amongst one or more computers. The processor 174 is in communication with the hardware interface 172, a user interface 176, and a memory 178. The hardware interface 172 is an interface which enables the processor 174 to send and receive data and/or commands to the rest of the medical instrument 100 and to control it. The user interface 176 may for example be a display and comprise a means for interacting with a graphical user interface rendered on the display. This may include the display being a touch screen and/or other entry device such as a mouse, trackball, or pad. The memory 178 may be any combination of volatile or non-volatile memory which the processor 174 has access to.

Machine-executable instructions 180 are shown as being stored in the memory 178. The machine-executable instructions enable the processor 174 to control the operation and function of the medical instrument 100. This includes control of the magnetic resonance imaging system 102 and the high-intensity focused ultrasound system 122.

The computer memory 178 is further shown as containing planning pulse sequence commands 182. The planning pulse sequence commands 182 are optional pulse sequence commands that may enable the processor 174 to acquire data for imaging which is then used to locate or target the sonication volumes 138, 138', 138". In some examples the planning pulse sequence commands 182 may also be used for acquiring calibration data for the magnetic resonance thermometry. The memory 178 is further shown as containing planning pulse sequence commands 184 that was acquired by controlling the magnetic resonance imaging system 102 with the planning pulse sequence commands 182. The memory 178 is shown as further containing one or more planning magnetic resonance images 186. The memory 178 is further shown as containing sonication commands 188. The sonication commands 188 could be obtained from a different machine via network, could be entered via the display 176 or user interface. In some instances they may be determined using the planning magnetic resonance image 186.

The memory 178 is further shown as containing an acoustic power setting 190 that was for example received from the user interface 176. The acoustic power setting 190 is a value which is used to scale the power delivered to individual transducer elements of the transducer 126. The memory is further shown as containing thermometry pulse sequence commands 192 which enables the processor 174 to acquire magnetic resonance thermometry data 193. The magnetic resonance thermometry data 193 is shown as being stored in the computer memory 178. The computer memory 178 is further shown as containing a thermal map 194 that has been constructed using the magnetic resonance thermometry data 193. The computer memory is further shown as containing a location for storing a state of a pause or resume command 196. For example when a pause command is received the sonication of the sonication volumes 138, 138', 138" may be paused. The memory 178 is further shown as containing a change power command 197.

The change power command 197 may be a command to change or rescale the acoustic power setting 190. The memory 178 is further shown as containing a predetermined value 198 which the change power command 197 is compared to. If the change power command 197 is greater than the predetermined value 198 and the sonication of the sonication volumes 138, 138', 138" is paused then a change in the acoustic power setting 190 is allowed. However, if the sonication of the volumes 138, 138', 138" is not paused and the change power command 197 is greater than the predetermined value 198 then the change is not allowed.

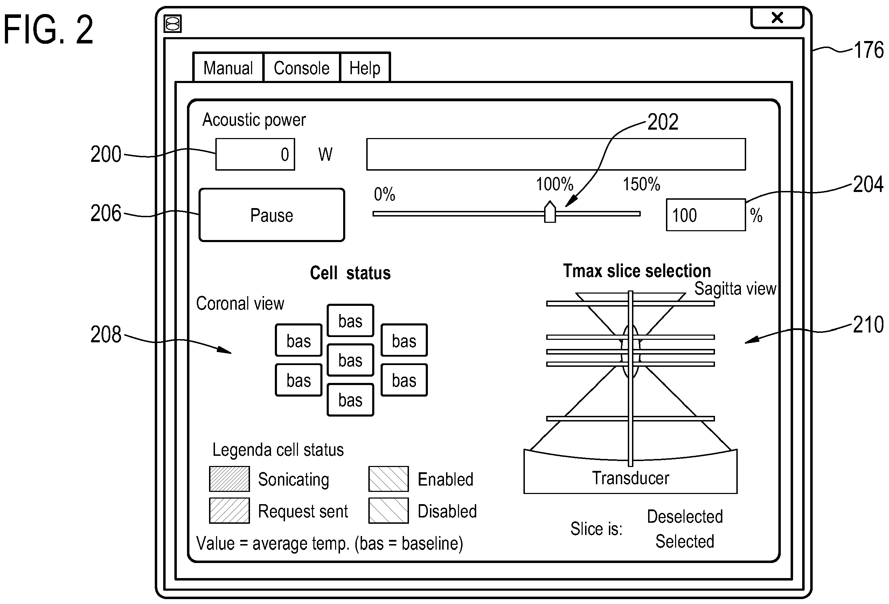

Fig. 2 shows an example of a graphical user interface 176 on a display. The graphical user interface has a box 200 for entering the acoustic power. The graphical user interface 176 further comprises a power adjustment slider 202 for receiving the change power command. The power adjustment slider 202 is an example of a power adjustment control. The graphical user interface 176 also comprises a box 204 that may either be used for generating the power adjustment numerically or for displaying the results of the power adjustment control 202. The user interface 176 further comprises a pause button 206. The button 206 may be used to either pause or resume the sonication of the sonication volumes. The user interface 176 is also shown as containing an optional sonication volume selection tool 208 and a slice selection tool 210.

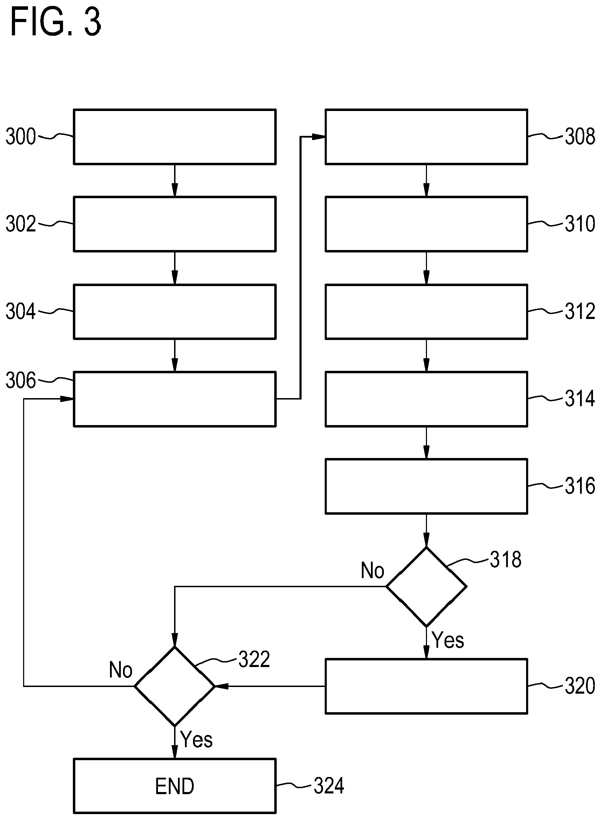

Fig. 3 shows a flowchart which illustrates a method of operating the medical instrument 100 of Fig. 1. First in step 300 the sonication commands 188 are received. Next in step 302 an acoustic power setting 190 is received. Next in step 304 the high-intensity focused ultrasound system 122 is controlled with the sonication commands 188. The acoustic power setting 190 is used to scale the power level of the sonication. In step 304 the sonication of the sonication volumes 138, 138', 138" is started. The method then proceeds to step 306. In step 306, the magnetic resonance imaging system 104 is controlled with the thermometry pulse sequence commands 192 to acquire the magnetic resonance thermometry data 193. Next in step 308 the thermal map 194 is reconstructed from the magnetic resonance thermometry data 193. The method steps after step 304 are part of a loop which is performed continuously during the sonication of the sonication volumes 138, 138', 138". Step 312 is the possible receiving of a pause command 196. If the pause command 196 has been received then the sonication of the sonication volumes 138, 138', 138" will be paused. Next in step 314 is the possible receiving of a resume command 196. If the resume command is received then a paused sonication will then start again.

Next step 316 is a possible reception of a change power command. The method then proceeds to step 318. Step 318 is a decision box in which a set of predetermined criteria can be used to determine if the power level of the acoustic power setting 190 should be changed or not. Depending upon different examples the criteria in step 318 may be different. For example, in one example if the change power command 197 is to increase the power at all the requirements in step 318 may require the sonication to be paused. In other examples the change power command 197 requires only that the power be below a predetermined value 198 to require the pausing of the sonication to be in effect. If the criteria for step 318 are fulfilled the method proceeds to step 320. In step 320, the value of the acoustic power setting 190 is changed and the power supplied to all the transducer elements of the transducer 126 are all scaled accordingly. If the criteria are not satisfied the method proceeds to step 322. In step 322 it is checked whether the sonication is still continuing or not. If the sonication ends then the method proceeds to step 324. If the sonication does not end the method proceeds back to step 306 where more magnetic resonance thermometry data 193 is acquired. This loop then continues until the sonication finishes 324.

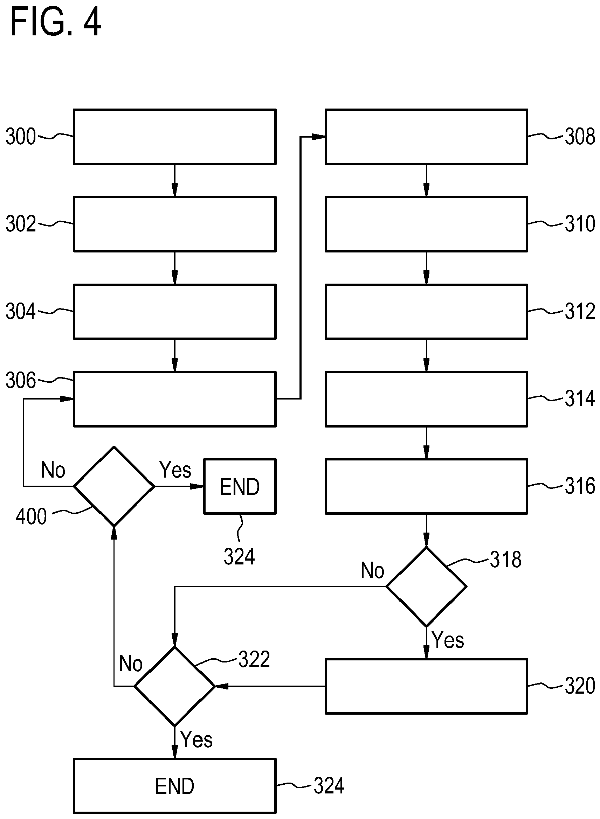

Fig. 4 shows a further example of a method of controlling the medical instrument 100 of Fig. 1. The method shown in Fig. 4 is similar to the method shown in Fig. 3. There is an additional decision step between step 322 and the return to the acquisition of the magnetic resonance thermometry data 193 in step 306. In step 400, any number of criteria can be checked which may result in the cancelling or abortion of the sonication of the volumes 138, 138', 138". For example if the pause in the sonication is longer than a predetermined delay, the temperature within the multiple sonication as determined by the thermal map drops below a predetermined lower threshold, the temperature within the multiple sonication volumes as determined by the thermal map is above a predetermined upper threshold, a temperature heating rate within one of the multiple sonication volumes is below a predetermined heating rate or a motion of the subject is detected may result in the sonication being cancelled. If any one of these or similar criteria are fulfilled the method proceeds to step 324 and the sonication ends. If none of these criteria are fulfilled the method proceeds back to step 306 and the magnetic resonance thermometry data 193 continues. It should be noted that in the method steps 3 and 4 the acquisition of the magnetic resonance thermometry data 193 and the generation of the thermal map 194 continues even if the sonication is paused. This may be useful in monitoring the state of the subject even though the sonication is paused.

During MR-HIFU hyperthermia treatment it may be beneficial to enable a higher acoustical power than the value which is set at the start of the therapy. This flexibility is required to prevent long delays when a therapy has to be stopped because hyperthermia could not be reached or maintained. Increasing the power creates an extra risk for the patient. Examples may have the benefit of mitigating this risk by forcing the treating physician to pause the sonication by pressing a pause button before he/she can increase the acoustic power level. After verification of the new level the sonication can be continued by deselecting the pause button.

Clinical studies show that hyperthermia can increase the efficacy of radiotherapy and chemotherapy for the treatment of specific malignant diseases (e.g. sarcoma, cervical cancer, etc.). Currently, only RF based systems are approved for the creation of hyperthermic temperatures in parts of the body. MR-HIFU can provide more conformal heating of the tumor to the hyperthermia temperature range, which should further boost the treatment efficacy. Examples may have the benefit of improving hyperthermia procedures in combined MRI and HIFU systems. Examples may provide for a Hyperthermia Manual Control (HMC) application that provides the treating physician more control over the MR-HIFU system during operation. It may help to prevent any long delays during therapy due to the cooling down time to baseline temperatures of the tissue after a hyperthermia session is aborted. When starting a new sonication, a new baseline temperature map is typically measured for which it may possibly be necessary that all tissue in the FOV is at body temperature. Within the HMC tool the acoustical power can be adjusted, selected treatment cells can be enabled or disabled, and one can determine in which slices the maximum allowable temperature (Tmax) should be monitored. An example of an HMC tool (graphical user interface) is illustrated in Fig. 2.

Prior to the start of the sonication the treating physician selects a power level. However, it is in practice very difficult to predict a power level that is high enough to induce sufficient heating, while being low enough that it does not induce temperature overshoots. The acoustical absorption of the tissue/tumor, which is unknown, will determine if the power is sufficient to reach hyperthermia in the target region. Also, dynamic patient-specific factors can have an effect on the target temperature during the therapy. It is well known that the tissue perfusion can increase when reaching elevated temperatures, and that edema formation can reduce absorption of ultrasonic waves in tissue. Both are difficult to predict, and can reduce the temperature during the therapy enough that it becomes problematic to keep the temperatures to the level of hyperthermia. To prevent temperature overshoots, causing e.g. vascular shutdown and/or pain, the acoustic power setting is limited due to the low temporal resolution of the temperature mapping sequence.

To reduce the number of times that the user needs to abort the treatment in order to find the fitting acoustical power, it is desirable to have the ability to adjust the power above or below the originally selected value during the treatment. However, this creates an extra risk in the system. The operator might unintentionally adjust the power to a level that can harm the patient or damage the tissue. Examples may help to reduce or mitigate this risk.

When the treating physician decides that the power is not sufficient, he can pause the sonication by selecting a pause button in the user interface, adjust the power above the preset value (100%) using a slider or other user interface, and, after verification, apply the new power setting by deselecting the pause button which restarts the sonication. During the pause the temperature maps may still be dynamically acquired.

Some examples may combine a functional combination of pause button and power slider (power adjustment slider) which offers increased treatment pace and safety while searching for the appropriate acoustic power during hyperthermia treatments. The increase in treatment pace may possibly be achieved by allowing the user to adjust the acoustic power level without aborting the sonication. The safety benefit is a direct result of this through the facilitated fine tuning of the treatment. Additional risks may possibly be mitigated by the forced use of the pause button before the power can be increased.

When a power level above the current maximum level is requested in the user interface (the user interface generates a change power command), the software may check if the sonication was paused (a pause command was received by the processor controlling the medical instrument). If not, the power will go to or stay at the current maximum level, and the user interface will indicate that the maximum power has been reached (for example, a slider controlling the power will go up to, but not exceed, the 100% mark). If the sonication is paused, the user interface allows power levels greater than 100% of the current level to be selected. When the operator clicks the pause button again to re-enable sonication, a command is sent to the HIFU controller to increase the power to the new level.

While the invention has been illustrated and described in detail in the drawings and foregoing description, such illustration and description are to be considered illustrative or exemplary and not restrictive; the invention is not limited to the disclosed embodiments.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measured cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

### LIST OF REFERENCE NUMERALS

- 100: medical instrument
- 102: magnetic resonance imaging system
- 104: magnet
- 106: bore of magnet
- 108: imaging zone
- 110: magnetic field gradient coils
- 112: magnetic field gradient coils power supply
- 114: radio-frequency coil
- 116: transceiver
- 118: subject
- 120: subject support
- 122: high intensity focused ultrasound system
- 124: fluid filled chamber
- 126: ultrasound transducer
- 128: mechanism
- 130: mechanical actuator/power supply
- 132: path of ultrasound
- 134: ultrasound window
- 136: gel pad
- 138: sonication volume
- 138': sonication volume
- 138": sonication volume
- 170: computer
- 172: hardware interface
- 174: processor
- 176: display / user interface
- 178: memory
- 180: machine executable instructions
- 182: planning pulse sequence commands
- 184: planning magnetic resonance data
- 186: planning magnetic resonance image
- 188: sonication commands
- 190: acoustic power setting
- 192: thermometry pulse sequence commands
- 193: magnetic resonance thermometry data
- 194: thermal map
- 196: pause or resume command
- 197: change power command
- 198: predetermined value
- 200: acoustic power entry box
- 202: power adjustment slider
- 204: power adjustment display / adjustment box
- 206: button
- 208: sonication volume selection tool
- 210: slice selection tool
- 300: receive sonication commands configured for controlling the ultrasonic transducer to sonicate the multiple sonication volumes
- 302: receive an acoustic power setting
- 304: control the high intensity focused ultrasound system with the sonication commands to sonicate the multiple sonication volumes;
- 306: control the magnetic resonance imaging system with the thermometry pulse sequence commands to acquire the magnetic resonance thermometry data
- 308: reconstruct a thermal map of the imaging zone using the magnetic resonance thermometry data;
- 310: display the thermal map on the display;
- 312: receive a pause command which causes the processor to pause the sonication of the multiple sonication volumes;
- 314: receive a resume command which causes the processor to resume the sonication of the multiple sonication volumes; and
- 316: receive a change power command which causes the processor to change the relative power of the electrical voltage supplied to each of the multiple ultrasonic transducer elements proportionally by a value specified in the change power command
- 318: Is sonication paused?
- 320: Change relative power level of the electrical power voltage supplied
- 322: Is sonication finished?
- 324: End sonication
- 400: Does criteria exist for aborting sonication?

## Claims

1. A medical instrument (100) comprising:
a magnetic resonance imaging system (102) for acquiring magnetic resonance thermometry data (193) from a subject (118) within an imaging zone (108);
a high intensity focused ultrasound system (122) comprising an ultrasonic transducer (126), wherein the ultrasonic transducer comprises multiple ultrasonic transducer elements, the ultrasonic transducer being configured for simultaneously heating multiple sonication volumes (138, 138', 138") within the imaging zone;
a memory (178) containing machine executable instructions (180) and thermometry pulse sequence commands (182), wherein the thermometry pulse sequence commands are configured for acquiring the magnetic resonance thermometry data according to a magnetic resonance imaging thermometry protocol;
a processor (174) for controlling the medical instrument; and
a display (176);
wherein the machine executable instructions cause the processor to:
receive (300) sonication commands (188) configured for controlling the ultrasonic transducer to sonicate the multiple sonication volumes, wherein the sonication commands are configured for controlling at least the relative power supplied to each of the multiple ultrasonic transducer elements;
receive (302) an acoustic power setting (190), wherein the relative power of the electrical voltage supplied to each of the multiple ultrasonic transducer elements is scaled using the acoustic power setting; and
control (304) the high intensity focused ultrasound system with the sonication commands to sonicate the multiple sonication volumes;
wherein execution of the machine executable instructions repeatedly cause the processor to perform the following during sonication of the multiple sonication volumes:
control (306) the magnetic resonance imaging system with the thermometry pulse sequence commands to acquire the magnetic resonance thermometry data;
reconstruct (308) a thermal map (194) of the imaging zone using the magnetic resonance thermometry data;
display (310) the thermal map on the display;
receive (312) a pause command (196) which causes the processor to pause the sonication of the multiple sonication volumes;
receive (314) a resume command (196) which causes the processor to resume the sonication of the multiple sonication volumes; and
receive (316) a change power command which causes the processor to change (320) the relative power of the electrical voltage supplied to each of the multiple ultrasonic transducer elements proportionally by a value specified in the change power command, wherein an increase of the relative power of the electrical voltage supplied to each of the multiple ultrasonic transducers elements above a predetermined value (198) is only performed if (318) the sonication of the multiple sonication volumes is paused.

2. The medical instrument of any one of the preceding claims, wherein execution of the machine executable instructions cause the processor to render a graphical user interface (176) on the display, wherein the graphical user interface comprises a power adjustment control (202) configured for generating the change power command.

3. The medical instrument of claim 2, wherein the graphical user interface further comprises at least one button (206) for generating the pause command and/or the resume command.

4. The medical instrument of any one of the preceding claims, wherein the high intensity focused ultrasound system is configured for performing hyperthermia within the multiple sonication volumes.

5. The medical instrument of any one of the preceding claims, wherein the high intensity focused ultrasound system is configured for heating the multiple sonication volumes within a predetermined temperature range.

6. The medical instrument of claim 5, wherein the predetermined temperature range is any one of the following:
between 40 degrees Celsius and 45 degrees Celsius and
between 41 degrees Celsius and 43 degrees Celsius.

7. The medical instrument of any one of the preceding claims, wherein
the predetermined value is the acoustic power setting.

8. The medical instrument of any one of claims 1 through 6, wherein the predetermined value is equivalent to a current acoustic power level supplied to each of the multiple ultrasonic transducers.

9. The medical instrument of any one of the preceding claims, wherein execution of the machine executable instructions cause aborting (400) of the sonication of the multiple sonication volumes if any one of the following is true:
a pause of the sonication is longer than a predetermined duration;
a temperature within the multiple sonication volumes as determined by the thermal map drops below a predetermined lower threshold;
the temperature within the multiple sonication volumes as determined by the thermal map rises above a predetermined upper threshold;
a temperature heating rate within one of the multiple sonication volumes is below a predetermined heating rate;
motion of the subject is detected and
combinations thereof.

10. The medical instrument of any one of the preceding claims, wherein one or more of the following operations by the processor are repeatedly performed during a pause of the sonication:
controlling the magnetic resonance imaging system with the thermometry pulse sequence commands to acquire the magnetic resonance thermometry data and reconstructing a thermal map of the imaging zone using the magnetic resonance thermometry data;
analyzing the thermal map for temperatures above a predetermined upper temperature limit, wherein the sonication is aborted if a temperature above the predetermined upper temperature limit is detected;
analyzing the thermal map to detect motion above a predetermined threshold, wherein the sonication if motion above the predetermined threshold is detected;
and combinations thereof.

11. A method of controlling a medical instrument (100), wherein the medical instrument comprises a magnetic resonance imaging system (102) for acquiring magnetic resonance thermometry data (193) from a subject (118) within an imaging zone (108), wherein the medical instrument further comprises a high intensity focused ultrasound system (122) comprising an ultrasonic transducer (126), wherein the ultrasonic transducer comprises multiple ultrasonic transducer elements, the ultrasonic transducer being configured for simultaneously heating multiple sonication volumes (138, 138', 138") within the imaging zone, wherein the medical instrument further comprises a display (176), wherein the method comprises:
receiving (300) sonication commands (188) configured for controlling the ultrasonic transducer to sonicate the multiple sonication volumes, wherein the sonication commands are configured for controlling at least the relative power supplied to each of the multiple ultrasonic transducer elements;
receiving (302) an acoustic power setting (190), wherein the relative power of the electrical voltage supplied to each of the multiple ultrasonic transducer elements is scaled using the acoustic power setting; and
controlling (304) the high intensity focused ultrasound system with the sonication commands to sonicate the multiple sonication volumes;
wherein the method further comprises performing the following during sonication of the multiple sonication volumes:
controlling (306) the magnetic resonance imaging system with thermometry pulse sequence commands to acquire magnetic resonance thermometry data, wherein the thermometry pulse sequence commands are configured for acquiring the magnetic resonance thermometry data according to a magnetic resonance imaging thermometry protocol;
reconstructing (308) a thermal map (194) of the imaging zone using the magnetic resonance thermometry data;
displaying (310) the thermal map on the display;
receiving (312) a pause command (196) which causes the processor to pause the sonication of the multiple sonication volumes;
receiving (314) a resume command (196) which causes the processor to resume the sonication of the multiple sonication volumes; and
receiving (316) a change power command (197) which causes the processor to change (320) the relative power of the electrical voltage supplied to each of the multiple ultrasonic transducer elements proportionally by a value specified in the change power command, wherein an increase of the relative power of the electrical voltage supplied to each of the multiple ultrasonic transducers elements above a predetermined value is only performed if (318) the sonication of the multiple sonication volumes is paused.

12. A computer program product comprising machine executable instructions (180) for execution by a processor (174) controlling a medical instrument (100), wherein the medical instrument comprises a magnetic resonance imaging system (102) for acquiring magnetic resonance thermometry data (193) from a subject (118) within an imaging zone (108), wherein the medical instrument further comprises a high intensity focused ultrasound system (122) comprising an ultrasonic transducer (126), wherein the ultrasonic transducer comprises multiple ultrasonic transducer elements, the ultrasonic transducer being configured for simultaneously heating multiple sonication volumes within the imaging zone, wherein the medical instrument further comprises a display, wherein execution of the machine executable instructions cause the processor to:
receive (300) sonication commands (188) configured for controlling the ultrasonic transducer to sonicate the multiple sonication volumes, wherein the sonication commands are configured for controlling at least the relative power supplied to each of the multiple ultrasonic transducer elements;
receive (302) an acoustic power setting (190), wherein the relative power of the electrical voltage supplied to each of the multiple ultrasonic transducer elements is scaled using the acoustic power setting; and
control (304) the high intensity focused ultrasound system with the sonication commands to sonicate the multiple sonication volumes;
wherein execution of the machine executable instructions repeatedly cause the processor to perform the following during sonication of the multiple sonication volumes:
control (306) the magnetic resonance imaging system with thermometry pulse sequence commands to acquire the magnetic resonance thermometry data, wherein the thermometry pulse sequence commands are configured for acquiring the magnetic resonance thermometry data according to a magnetic resonance imaging thermometry protocol;
reconstruct (308) a thermal map (194) of the imaging zone using the magnetic resonance thermometry data;
display (310) the thermal map on the display;
receive (312) a pause command (196) which causes the processor to pause the sonication of the multiple sonication volumes;
receive (314) a resume command (196) which causes the processor to resume the sonication of the multiple sonication volumes; and
receive (316) a change power command (197) which causes the processor to change (320) the relative power of the electrical voltage supplied to each of the multiple ultrasonic transducer elements proportionally by a value specified in the change power command, wherein an increase of the relative power of the electrical voltage supplied to each of the multiple ultrasonic transducers elements above a predetermined value is only performed if (320) the sonication of the multiple sonication volumes is paused.

13. The computer program product of claim 12, wherein execution of the machine executable instructions further cause the processor to render a graphical user interface (176) on the display, and wherein the graphical user interface comprises a power adjustment control(202) configured for generating the change power command.

14. The computer program product of claim 12 or 13, wherein the graphical user interface further comprises at least one button (206) for generating the pause command and/or the resume command.
